# FASCICULE DE BREVET EUROPEEN

(11) **EP 1 349 919 B1**
(45) Date de publication et mention de la délivrance du brevet: **03.07.2013**
(21) Numéro de dépôt: 02711933.8
(22) Date de dépôt: 10.01.2002
(51) Int. Cl.: C12N 5/0775, A61K 35/12, A61P 7/00, A61P 37/00, A61P 21/00, A61P 9/10

(54) **CELLULES DU TISSU ADIPEUX EXTRAMEDULLAIRE ET LEURS APPLICATIONS DANS LA RECONSTITUTION DU TISSU CARDIAQUE**
ZELLEN AUS EXTRAMEDULLÄREM FETTGEWEBE UND DEREN ANWENDUNGEN ZUR WIEDERHERSTELLUNG VON HARTZGEWEBE
EXTRAMEDULLARY ADIPOSE TISSUE CELLS AND USE THEREOF FOR REGENERATING CARDIAC TISSUE

(30) Priorité: 10.01.2001 FR 0100249
(43) Date de publication de la demande: 08.10.2003
(73) Titulaire: CENTRE NATIONAL DE LA RECHERCHE SCIENTIFIQUE (CNRS), 75794 Paris Cedex 16 (FR); INSTITUT NATIONAL DE LA SANTE ET DE LA RECHERCHE MEDICALE, 75654 Paris Cédex 13 (FR)
(72) Inventeur: CASTEILLA, Louis, F-31750 Escalouens (FR); COUSIN, Béatrice, F-31860 Pins Justaret (FR); PENICAUD, Luc, F-31400 Toulouse (FR); ANDR , Mireille, F-31700 Blagnac (FR)
(74) Mandataire: Orès, Bernard
(86) Numéro de dépôt international: PCT/FR2002/000071
(87) Numéro de publication internationale: WO 2002/055678

(56) Documents cités:
- EP-A- 1 077 254
- WO-A-00/44882
- WO-A-01/62901
- WO-A-99/02654
- WO-A-99/28444
- BJÖRNTORP P ET AL: "Isolation and characterization of cells from rat adipose tissue developing into adipocytes" JOURNAL OF LIPID RESEARCH, vol. 19, no. 3, 1978, pages 316-324, XP000613823 cité dans la demande
- JACKSON K A ET AL: "Hematopoietic potential of stem cells isolated from murine skeletal muscle" PROCEEDINGS OF THE NATIONAL ACADEMY OF SCIENCES OF USA, vol. 96, no. 25, 7 décembre 1999 (1999-12-07), pages 14482-14486, XP002161311 cité dans la demande
- ZUK P A ET AL: "Multilineage cells from human adipose tissue: Implications for cell-based therapies." TISSUE ENGINEERING, vol. 7, no. 2, avril 2001 (2001-04), pages 211-228, XP002198710 cité dans la demande
- MALOUF N N ET AL: "Adult-derived stem cells from the liver become myocytes in the heart in vivo." AMERICAN JOURNAL OF PATHOLOGY, vol. 158, no. 6, juin 2001 (2001-06), pages 1929-1935, XP008003219
- STOCUM D L: "Regeneration biology and engineering: Strategies for tissue restoration." WOUND REPAIR AND REGENERATION, vol. 6, no. 4, juillet 1998 (1998-07), pages 276-290, XP002907218
- DANI C ET AL: 'Cloning and regulation of a mRNA specifically expressed in the preadipose state.' JOURNAL OF BIOLOGICAL CHEMISTRY vol. 264, no. 17, 15 Juin 1989, pages 10119 - 10125, XP055033820 ISSN: 0021-9258
- SUL HS: "Minireview: Pref-1: Role in Adipogenesis and Mesenchymal Cell Fate", MOLECULAR ENDOCRINOLOGY, vol. 23, no. 11, November 2009 (2009-11), pages 1717-1725, XP002622832, ISSN: 0888-8809, DOI: 10.1210/ME.2009-0160 [retrieved on 2009-06-18]
- PLANAT-BÉNARD V ET AL: "Spontaneous cardiomyocyte differentiation from adipose tissue stroma cells.", CIRCULATION RESEARCH, vol. 94, no. 2, 6 February 2004 (2004-02-06), pages 223-229, ISSN: 1524-4571
- LÉOBON B ET AL: "Adipose-derived cardiomyogenic cells: in vitro expansion and functional improvement in a mouse model of myocardial infarction.", CARDIOVASCULAR RESEARCH, vol. 83, no. 4, September 2009 (2009-09), pages 757-767, ISSN: 1755-3245

## Description

La présente invention est relative à des cellules issues du tissu adipeux extra-médullaire, à leurs procédés de préparation, ainsi qu'à leurs applications dans la reconstruction musculaire cardiaque, notamment pour le traitement des myopathies, des cardiomyopathies et des maladies liées à une dégénérescence musculaire (infarctus du myocarde).

La reconstruction du tissu musculaire cardiaque (myocarde) a été envisagée assez récemment étant donné que contrairement aux muscles squelettiques, le coeur ne possède pas de réservoirs de cellules précurseurs. Pour reconstruire le myocarde, il été proposé de greffer des cellules satellites autologues dans le coeur. Toutefois, cette approche n'est pas satisfaisante dans la mesure où ces cellules satellites greffées se différencient en cellules musculaires squelettiques qui n'ont pas les mêmes caractéristiques contractiles que les cardiomyocytes.

Par conséquent, il existe un réel besoin de disposer de nouveaux moyens et notamment de cellules aptes à à reconstruire le myocarde, qui soient plus efficaces et plus simples à mettre en oeuvre que les moyens de l'art antérieur.

Il a récemment été montré que des cellules souches de certains tissus non-hématopoïétiques étaient capables de reconstituer l'ensemble des lignées hématopoïétiques de souris, irradiées de façon létale, à savoir :
- des cellules souches musculaires issues du muscle squelettique de souris adultes exprimant :
   - soit des marqueurs communs à toutes les cellules souches (Sca-1, c-Kit) mais pas le marqueur CD45, qui est spécifique des cellules souches hématopoïétiques (Jackson et al., PNAS, 1999, 96, 14482-14486),
   - soit le récepteur de la protéine morphogénique du tissu osseux de type 2 (BMP2), (Pang, Blood, 2000, 95, 1106-1108), et
   - des cellules souches neurales issues du cerveau de souris adulte (Bjornson et al., Science, 1999, 283, 534-537).

De façon analogue, pour obtenir des cardiomyocytes, il a été proposé d'utiliser des cellules embryonnaires humaines, des cellules mésenchymateuses de la moelle osseuse ou des cellules endothéliales (Kehat et al., J. Clin. Invest., 2001, 108, 407-414 ; Muller et al., FASEB J., 2000, 14, 2540-2548, Toma et al., Circulation, 2002, 105, 93-98 ; Liechty et al., Nat. Medicine, 2000, 11, 1282-1286 ; Condorelli et al., PNAS, 2001, 98, 10733-10738 ; Wang et al., J. Thorac. Cardiovasc. Surg 2001, 122, 699-705 ; Jackson et al., J. Clin. Invest., 2001, 107, 1395-1402).

Deux hypothèses ont été émises pour expliquer ces résultats : des cellules souches proches de la totipotence des cellules embryonnaires persisteraient dans des tissus de l'adulte (cerveau, muscle..) et seraient capables de se différencier en différents types cellulaires, ou bien des cellules souches spécialisées de ces tissus possèderaient une très grande plasticité et seraient capables de se dé-différencier ou d'être reprogrammées (transdifférenciation).

Ces résultats ont des conséquences importantes pour le traitement des déficits fonctionnels de la moelle osseuse (aplasie médullaire), le traitement de la contamination de la moelle osseuse par des cellules tumorales (neuroblastome) et pour la correction d'anomalies génétiques des cellules hématopoïétiques (manipulation génétique du tissu hématopoïétique). Ces résultats ont également des conséquences pour le traitement des déficits musculaires fonctionnels (myopathies et cardiomyopathies) et des maladies liées à une dégénérescence musculaire (infarctus du myocarde).

En effet, l'utilisation de cellules souches ou de précurseurs de tissus non-hématopoïétiques permettrait d'éviter les problèmes liés au rejet de greffe de moelle osseuse ou à une quantité insuffisante de cellules CD34, dans la mesure où l'on pourrait envisager la reconstitution des lignées hématopoïétiques d'un individu adulte malade, par greffe de tissu nerveux ou musculaire autologue, prélevé chez ce même individu. De même, l'utilisation de cellules souches autres que les cellules satellites, permettrait de reconstruire le myocarde par greffe des cellules de moelle osseuse, des cellules embryonnaires ou des cellules endothéliales.

Cependant, en pratique, la reconstruction du tissu musculaire cardiaque à partir des cellules précitées, est difficilement réalisable en raison des difficultés techniques de prélèvement et des faibles quantités de tissus disponibles. A ces difficultés techniques, s'ajoutent également des problèmes éthiques liés à l'utilisation de tissus embryonnaires.

Par conséquent, les Inventeurs se sont donnés pour but de pourvoir à des cellules aptes à reconstituer les tissus musculaires de façon durable, qui soient isolées à partir de tissus faciles à prélever et disponibles en quantités importantes.

Le tissu adipeux existe sous différentes formes chez les mammifères : le tissu adipeux blanc qui représente le principal organe de réserve de l'organisme, le tissu adipeux brun thermogénique et le tissu adipeux médullaire dont le rôle exact n'est pas connu.

Ce tissu adipeux est constitué par deux fractions cellulaires :
- une fraction adipocytaire comprenant des cellules adipeuses différenciées : adipocytes immatures (petites cellules adipeuses) et adipocytes matures qui représentent 30 % à 60 % des cellules du tissu adipeux. Cette fraction cellulaire est caractérisée par l'accumulation de triglycérides et l'expression de marqueurs tardifs et très tardifs comme la GPDH (glycérol-3-phosphate déshydrogénase), et
- une fraction non-adipocytaire, appelée fraction du stroma vasculaire comprenant quelques cellules sanguines, endothéliales, des péricytes, des fibroblastes et des précurseurs adipocytaires, en particulier des préadipocytes caractérisés par l'absence de lipides dans leur cytoplasme et l'expression de marqueurs précoces comme la chaîne *α*2 du collagène VI (A2COL6/pOb24) et la lipoprotéine lipase (LPL).

Ces deux fractions cellulaires peuvent être séparées par leur différence de densité, selon des procédés tels que ceux décrits par Björntorp et al. (J. Lipid Res., 1978, 19, 316-324).

De façon classique, la différenciation des adipocytes est schématisée de la façon suivante : cellule souche multipotente -> précurseur adipocytaire ou préadipocyte -> adipocyte immature -> adipocyte mature.

Les précurseurs très précoces, à savoir des cellules souches multipotentes capables d'engendrer différents types de cellules mésenchymateuses comme des cellules adipocytaires et des cellules musculaires n'ont pas été identifiées. Toutefois, il a été montré que des lignées de cellules mésenchymateuses étaient capables de se différencier en adipocytes, chondrocytes ou en fibroblastes, spontanément (lignée de tératocarcinome T984) ou après traitement avec de la 5-azacytidine (10T1/2, 3T3, CHEF-18).

Des précurseurs adipocytaires ont été clonés à partir de ces lignées (lignée clonale 1246), à partir d'embryon de souris (3T3-L1, 3T3-F442A, A31T, TA1) ou de hamster (CHEF-18) ou bien à partir de souris adulte (Ob17, HFGu, BFC-1, ST13, MS3-2A, MC 3t3-G2/PA6). Ces précurseurs adipocytaires sont capables de se différencier en adipocytes, et ce *in vitro* ou *in vivo* (Ailhaud et al., Annu. Rev. Nutr., 1992, 12, 207-233).

En outre, dans des travaux antérieurs (Cousin et al., The FASEB Journal, 1999, 13, 305-312), les Inventeurs qui se sont intéressés à la relation entre les adipocytes et les macrophages dans l'obésité et la réponse inflammatoire ont montré que les préadipocytes (cultures primaires issues de la fraction du stroma vasculaire ou lignée 3T3-L1), comme des macrophages, possèdent une activité de phagocytose et que le marqueur MOMA-2 qui est spécifique des monocytes-macrophages est également exprimé par les préadipocytes et les adipocytes. Toutefois, il ressort de cet article que les préadipocytes sont différents des macrophages dans la mesure où par des techniques classiques, il est impossible de détecter la présence, à la surface de ces cellules, des marqueurs F4/80 et Mac1 qui sont spécifiques des macrophages matures.

Le tissu adipeux a un potentiel de régénération qui persiste pendant toute la vie de l'individu et est lié au maintien d'une population de préadipocytes, au sein des différents dépôts adipeux. En effet, le nombre d'adipocytes présents dans un dépôt donné peut varier dans des proportions considérables selon les conditions physiologiques ou physiopathologiques.

Ainsi, il a été montré que chez l'homme ou chez les rongeurs adultes, même âgés, les cellules de la fraction du stroma vasculaire du tissu adipeux qui comprennent une proportion importante de préadipocytes exprimant des marqueurs précoces de différenciation (A2COL6/pOb24, LPL, IGF-1....) sont capables de proliférer *in vitro* et de se différencier en adipocytes (Ailhaud et al., 1992, précité).

Ainsi, il a été proposé d'utiliser des cellules souches multipotentes issues de la fraction du stroma vasculaire du tissu adipeux pour reconstituer des lignées hématopoïétiques, ainsi que des tissus nerveux et hépatiques (Demande Internationale WO 01/62901) ou des tissus musculaires, osseux et cartilagineux (Zuk et al., Tissue Eng., 2001, 7, 20, 211-228). Toutefois, les moyens décrits dans ces documents n'ont pas permis d'isoler de telles cellules souches effectivement aptes à se différencier en cellules fonctionnelles capables de reconstituer une activité musculaire cardiaque.

De manière surprenante, les Inventeurs ont maintenant montré que des cellules de la fraction du stroma vasculaire du tissu adipeux extra-médullaire (ou fraction cellulaire du stroma vasculaire) sont effectivement capables de se différencier en cardiomyocytes; de telles cellules sont aptes à reconstituer un coeur fonctionnel, notamment lorsqu'elles sont greffées dans la zone d'infarcie, après un accident cardiaque.

La présente invention a, en conséquence, pour objet la fraction cellulaire du stroma vasculaire du tissu adipeux extra-médullaire comme médicament ; en effet, cette fraction permet, de manière surprenante la reconstruction du tissu musculaire cardiaque (myocarde).

L'invention a également pour objet l'utilisation de ladite fraction pour la préparation d'un médicament destiné au traitement des myopathies et des cardiomyopathies d'origine génétique ou acquise, et des pathologies (induites ou constitutives), liées à une dégénérescence musculaire telles que l'infarctus du myocarde.

En effet, des cellules de la fraction du stroma vasculaire du tissu adipeux extra-médullaire sont capables de se différencier en cardiomyocytes fonctionnels, présentant une activité contractile.

Conformément à l'invention, la fraction cellulaire du stroma vasculaire est isolée par différence de densité, notamment selon le protocole décrit par Björntorp et al. (précité).

La présente invention a également pour objet des cellules isolées et purifiées, aptes à se différencier en cardiomyocytes, caractérisées en ce qu'elles sont susceptibles d'être obtenues par les étapes successives suivantes :
- prélèvement d'un échantillon de tissu adipeux extra-médullaire,
- isolement de la fraction cellulaire du stroma vasculaire, de préférence, par digestion de la matrice extracellulaire par des enzymes protéolytiques, et par séparation physique, notamment par différence de densité, et
- purification des cellules par séparation physique et/ou par immunosélection, et en ce qu'elles expriment au moins un marqueur des cellules souches ou des précurseurs adipocytaires sélectionné dans le groupe constitué par A2COL6/pOb24 et Pref-1 et au moins un marqueur des précurseurs cardiomyocytaires sélectionné dans le groupe constitué par l'α-actinine et le facteur GATA-4.

Avantageusement :
- la séparation physique est réalisée par différence d'adhésion sur un support solide approprié ou par différence de densité (centrifugation dans un gradient approprié, élutriation),
- l'immunosélection est réalisée à l'aide d'au moins un anticorps spécifique d'un marqueur exprimé par les précurseurs adipocytaires, hématopoïétiques ou cardiomyocytaires (sélection positive) et/ou d'au moins un anticorps spécifique d'un marqueur absent desdits précurseurs (sélection négative), qui sont connus en eux même de l'Homme du métier.

Selon une disposition avantageuse de ce mode de réalisation, l'étape de purification est précédée par une étape supplémentaire de culture des cellules dans un milieu semi-solide contenant des facteurs de croissance et/ou des cytokines appropriées.

A titre d'exemple non-limitatif, on peut citer un milieu contenant de la méthylcellulose additionnée de sérum de veau foetal, de sérum de boeuf, d'insuline, de transferrine, de SCF (*Stem Cell Factor*), d'IL3 et d'IL6.

Dans le cadre de l'invention, les cellules souches et les précurseurs correspondent à des cellules multipotentes qui possèdent des propriétés d'expansion clonale et de différenciation tissulaire, et ces deux termes sont considérés comme équivalents.

De préférence, lesdites cellules telles que définies ci-dessus sont d'origine humaine.

La présente invention a également pour objet des cellules modifiées, caractérisées en ce qu'elles sont constituées par des cellules telles que définies ci-dessus génétiquement modifiées.

Selon un mode de réalisation avantageux desdites cellules modifiées, elles comprennent au moins une mutation d'un gène autologue.

Au sens de la présente invention, on entend par mutation d'un gène, une insertion, une délétion ou une substitution d'au moins un nucléotide dudit gène.

Par exemple, des gènes du CMH desdites cellules peuvent être mutés, afin de permettre une greffe hétérologue.

Selon un autre mode de réalisation desdites cellules modifiées, elles contiennent au moins une copie d'un gène hétérologue, notamment un gène d'intérêt thérapeutique. Avantageusement, le produit dudit gène est sécrété par lesdites cellules modifiées.

Par exemple, lesdites cellules expriment une interleukine ou un facteur agissant sur la coagulation sanguine.

Conformément à l'invention, lesdites cellules modifiées sont obtenues selon des techniques qui sont connues en elles-mêmes de l'Homme du métier ; on peut citer notamment la recombinaison homologue, l'infection par un vecteur recombinant tel qu'un virus recombinant (rétrovirus, lentivirus, adénovirus ou virus associé à l'adénovirus (AAV)) ou la transfection par un plasmide recombinant, qui sont décrites dans Current Protocols in Molecular Biology, (1990-2000), John Wiley and Sons, Inc. New York. Selon la nature du vecteur recombinant, ledit gène hétérologue d'intérêt est intégré dans le génome desdites cellules ou bien il est présent sous forme extra-chromosomique.

De préférence, lesdites cellules modifiées telles que définies ci-dessus sont d'origine humaine.

La présente invention a également pour objet des lignées de cellules immortalisées issues des cellules humaines telles que définies ci-dessus.

Conformément à l'invention, les lignées de cellules immortalisées sont obtenues par passages successifs, comme décrit dans Green et al., Cell, 1974, 3, 127-133.

De manière avantageuse, ledit médicament est administré par voie parentérale, de préférence par voie intraveineuse.

La présente invention a également pour objet un médicament destiné à la reconstruction du myocarde, caractérisé en ce qu'il comprend des cellules (isolées et/ou modifiées) aptes à se différencier en cardiomyocytes ou des lignées issues de ces cellules telles que définies ci-dessus et au moins un véhicule pharmaceutiquement acceptable.

De manière avantageuse, ledit médicament est administré, localement au site de la lésion.

La présente invention a également pour objet l'utilisation des cellules aptes à se différencier en cardiomyocytes ou bien des cellules modifiées ou des lignées issues de ces cellules, telles que définies ci-dessus, pour la préparation d'un médicament destiné au traitement des cardiomyopathies et des maladies dans lesquelles on observe une dégénérescence musculaire cardiaque.

La présente invention a également pour objet l'utilisation des cellules aptes à se différencier en cardiomyocytes ou bien des cellules modifiées ou des lignées issues de ces cellules, telles que définies ci-dessus, pour le criblage de molécules capables de moduler (activer ou inhiber) l'activité cardiaque.

La présente invention a également pour objet un procédé de préparation des cellules isolées et purifiées aptes à se différencier en cardiomyocytes, telles que définies ci-dessus, lequel procédé est caractérisé en ce qu'il comprend au moins les étapes suivantes :
a₂) prélèvement d'un échantillon de tissu adipeux extra-médullaire,
b₂) isolement de la fraction cellulaire du stroma vasculaire, de préférence par digestion de la matrice extracellulaire par des enzymes protéolytiques, et par séparation physique, et
c₂) purification des cellules par séparation physique et/ou par immunosélection.

Selon un mode de mise en oeuvre avantageux dudit procédé, préalablement à l'étape c₁ ou c₂, il comprent une étape supplémentaire de culture des cellules dans un milieu semi-solide contenant des facteurs de croissance et/ou des cytokines appropriées.

Selon un mode de mise en oeuvre avantageux dudit procédé, ils comprent une étape supplémentaire d₁), d₂) ou e₃) d'expansion des cellules *in vitro.*

Avantageusement, la séparation physique est réalisée par différence d'adhésion sur un support solide ou par différence de densité et l'immunosélection est réalisée à l'aide d'au moins un anticorps spécifique d'un marqueur exprimé par lesdites cellules (sélection positive) et/ou d'au moins un anticorps spécifique d'un marqueur absent desdites cellules (sélection négative) tels que définis ci-dessus.

De manière avantageuse, pour la mise en oeuvre du procédé d'obtention des cellules isolées et purifiées selon l'invention :
- le prélèvement (étapes a₁, a₂ ou a₃) peut-être effectué au niveau d'un dépôt adipeux facilement accessible, comme un dépôt adipeux sous-cutané,
- la fraction cellulaire du stroma vasculaire est isolée (étape b₁, b₂ ou b₃) par différence de densité, notamment selon le protocole décrit par Björntorp et al. (précité),
- la culture des cellules dans un milieu semi-solide contenant des facteurs de croissance et/ou des cytokines appropriées (étapes supplémentaires ou étape c₃) est réalisée dans un milieu contenant de la méthylcellulose additionnée de sérum de veau foetal, de sérum de boeuf, d'insuline, de transferrine, de SCF, d'IL3 et d'IL6.
- la purification des cellules (étape c₁, c₂ ou d₃) est réalisée soit par séparation sur tout support approprié (différence d'adhésion) ou bien par centrifugation dans un gradient approprié ou par élutriation (différence de densité), soit par immunosélection, selon les techniques classiques d'immunocytochimie, notamment les techniques de tri (positif ou négatif) de cellules immunomarquées en cytométrie de flux ou à l'aide de billes magnétiques, telles que décrites par exemple dans Current protocols in Immunology (John E. Coligan, 2000, Wiley and son Inc, Library of Congress, USA*) ;* au moins un anticorps spécifique d'un marqueur exprimé par lesdites cellules (sélection positive) et/ou au moins un anticorps spécifique d'un marqueur absent desdites cellules (sélection négative) tels que définis ci-dessus sont utilisés pour le tri des cellules.
- l'expansion des cellules *in vitro* (étape d₁, d₂ ou e₃) est réalisée dans un milieu de culture convenable, tel que par exemple, mais de manière non limitative, un milieu DMEM F12 comprenant soit du sérum de veau foetal, soit un substitut végétal du sérum.

Par rapport aux moyens existants de reconstruction du tissus musculaire cardiaque, les fractions cellulaires et les cellules isolées, ainsi que leurs procédés de préparation, tels que définis ci-dessus présentent les avantages suivants :
- avantages techniques
   - facilité de prélèvement,
   - quantité de tissu et de cellules très importante avec expansion possible des cellules prélevées, favorable à une greffe homologue ou hétérologue,
   - possibilité de maintenir et de multiplier, voire d'immortaliser, les cellules *in vitro* dans un milieu défini, favorable à une greffe homologue ou hétérologue,
   - possibilité de reconstituer le tissu musculaire de plusieurs individus à partir d'un seul individu,
   - possibilité de maintenir les cellules congelées,
   - cellules transfectables,
   - cellules à fort pouvoir sécréteur qui pourront être utilisées pour libérer des protéines d'intérêt thérapeutique,
   - cellules adaptées au criblage *in vitro,* d'une quantité importante de molécules thérapeutiques susceptibles de moduler l'activité musculaire cardiaque.
- avantages économiques :
   - temps d'hospitalisation réduit (pas de conditionnement ni de cytaphérèse)
- avantages éthiques :
   - prélèvements peu invasifs,
   - absence d'utilisation de tissus embryonnaires.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :

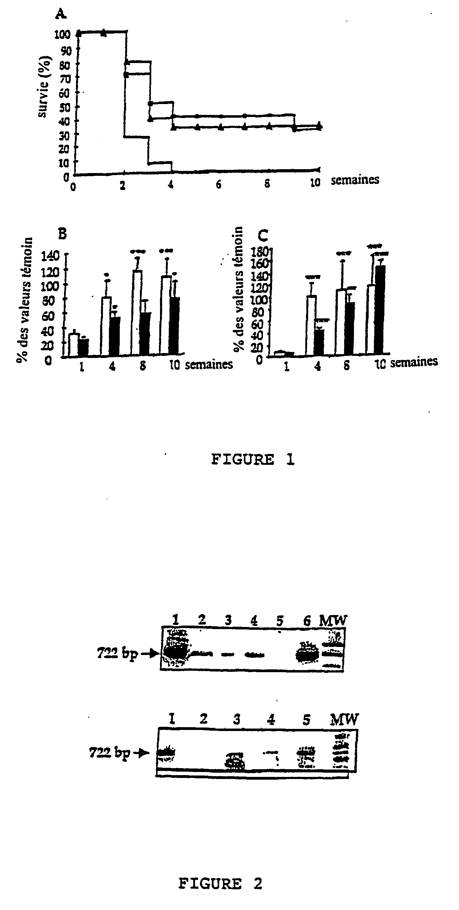

- la figure 1 illustre la reconstitution des lignées hématopoïétiques, obtenue par injection de cellules du stroma vasculaire du tissu adipeux extra-médullaire ou de cellules de moelle osseuse (contrôle). La figure 1A est un graphique de Kaplan-Meier représentant le pourcentage de survie des souris irradiées de façon létale (en ordonnée), sur une durée de 10 semaines suivant l'irradiation (en abscisse). Les souris irradiées non-reconstituées sont représentées par des cercles, les souris transplantées par des cellules de moelle osseuses sont représentées par des carrés et les souris transplantées par des cellules du stroma vasculaire sont représentées par des triangles. Chaque groupe comprend un nombre initial de 10 à 15 souris. Les figures 1B et 1C illustrent respectivement le nombre de plaquettes et de leucocytes chez des souris irradiées reconstituées avec des cellules de moelle osseuse (en noir) ou des cellules du stroma vasculaire du tissu adipeux (en blanc). Les résultats sont exprimés en pourcentage par rapport aux valeurs des témoins non-irradiés, et les valeurs indiquées représentent la moyenne ± erreur standard, obtenue sur des groupes de 5 à 15 souris.
- la figure 2 illustre la détection par PCR du gène *sry* spécifique du chromosome Y dans la rate (panneau supérieur) et le sang (panneau inférieur) des souris femelles reconstituées, effectuée 10 semaines après la transplantation de cellules de moelle osseuse ou de cellules du stroma vasculaire. Panneau supérieur : la PCR est effectuée sur 50 ng (lignes 1 ; 5 et 6) ou 150 ng (lignes 2 à 4) d'ADN de rate. Un produit de 722 pb est détecté chez les souris reconstituées avec des cellules de moelle osseuse (ligne 1) ou du stroma vasculaire (lignes 2-4), issues d'une souris mâle. Aucun signal n'est détecté chez les souris femelles témoin (ligne 5). Un échantillon de sang d'une souris mâle est utilisé comme témoin positif (ligne 6). Un marqueur de poids moléculaire est indiqué en référence (MW). Panneau inférieur : la PCR est réalisée sur 50 ng d'ADN de sang. Un produit de 722 pb est détecté chez les animaux reconstitués avec des cellules de moelle osseuse (ligne 5) ou du stroma vasculaire (lignes (3-4), issues d'une souris mâle. Aucun signal n'est détecté chez les souris femelles témoin (ligne 2). Un échantillon de sang d'une souris mâle est utilisé comme témoin positif (ligne 1). Un marqueur de poids moléculaire est indiqué en référence (MW).

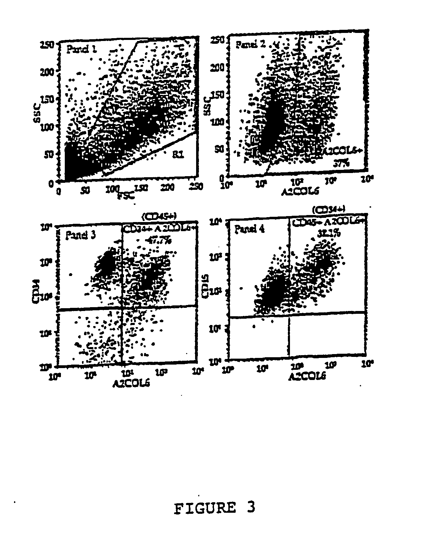

- la figure 3 illustre l'analyse en cytométrie de flux des cellules du stroma vasculaire des souris C57B1/6 mâles. Panneau 1 : la région R1 correspond à la population cellulaire sélectionnée pour l'analyse, en fonction des paramètres de granulométrie (FSC : *Forwards Scatter),* en abscisse et des paramètres de taille (SSC : *Side Scatter*), en ordonnée. Panneau 2 : répartition des cellules positives pour l'antigène A2COL6 spécifique des préadipocytes (en abscisse), en fonction de la taille des cellules (en ordonnée). Panneaux 3 et 4 : représentation d'un triple marquage pour l'antigène spécifique des préadipocytes (A2COL6) et pour deux antigènes spécifiques des cellules souches hématopoïétiques (CD45 et CD34). Le panneau 3 représente la répartition des cellules A2COL6⁺ (en abscisse) et CD34⁺ (en ordonnée) dans la population de cellules CD45⁺. Le panneau 4 représente la répartition des cellules A2COL6⁺ (en abscisse) et CD45⁺ (en ordonnée) dans la population de cellules CD34⁺

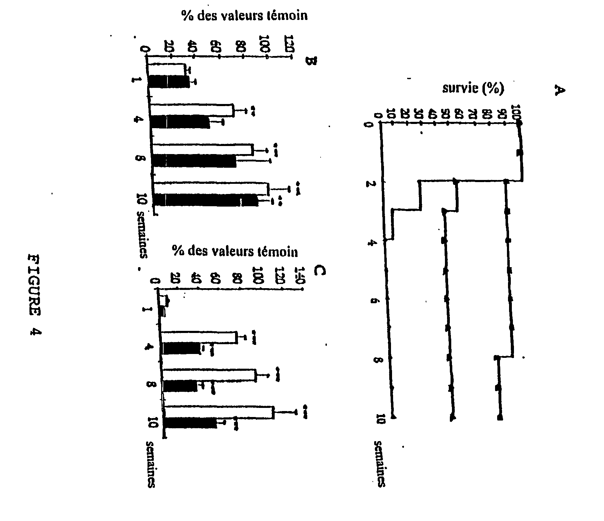

- la figure 4 illustre la reconstitution des lignées hématopoïétiques obtenue par injection de la lignée préadipocytaire 3T3-L1 ou de cellules de moelle osseuse (contrôle). La figure 1A est un graphique de Kaplan-Meier représentant le pourcentage de survie des souris irradiées de façon létale (en ordonnée), sur une durée de 10 semaines suivant l'irradiation (en abscisse). Les souris irradiées non-reconstituées sont représentées par des cercles, les souris transplantées par des cellules de moelle osseuses par des carrés et les souris transplantées par la lignée préadipocytaire 3T3-L1 par des triangles. Chaque groupe comprend un nombre initial de 10 à 15 souris. Les figures 4B et 4C représentent respectivement le nombre de plaquettes et de leucocytes chez des souris irradiées reconstituées avec des cellules de moelle osseuse (en blanc) ou avec la lignée préadipocytaire 3T3-L1 (en noir). Les résultats sont exprimés en pourcentage par rapport aux valeurs des témoins non-irradiés, et les valeurs indiquées représentent la moyenne ± erreur standard, obtenue sur des groupes de 5 à 15 souris.

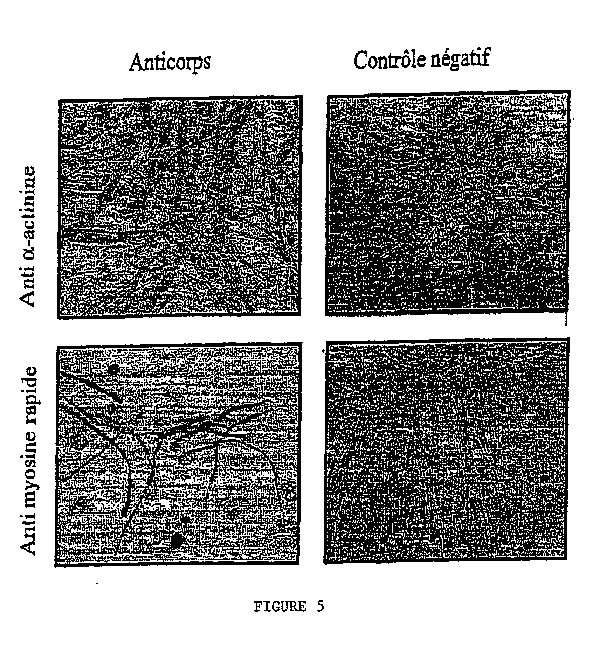

- la figure 5 illustre l'analyse par immunocytochimie de la différenciation en cardiomyocytes et en cellules musculaires squelettiques des cellules isolées à partir du stroma vasculaire du tissu adipeux extra-médullaire, selon les procédés de l'invention. Panneaux supérieurs : la présence de cardiomyocytes et de cellules musculaires squelettiques différenciées est détectée spécifiquement à l'aide d'un anticorps anti α-actinine (panneau de gauche), par comparaison, dans le contrôle négatif, aucun marquage n'est observé en l'absence d'anticorps anti α-actinine (panneau de droite). Panneaux inférieurs : la présence de cellules musculaires squelettiques différenciées est détectée spécifiquement à l'aide d'un anticorps anti-isoformes rapides de la myosine (panneau de gauche), par comparaison, dans le contrôle négatif, aucun marquage n'est observé en l'absence d'anticorps anti-isoformes rapides de la myosine α- (panneau de droite).

### EXEMPLE 1 Matériels et méthodes.

### 1) Isolement de cellules de moelle osseuse

Les cellules de moelle osseuse sont isolées à partir de fémurs de souris C57B1/6 mâles âgées de 6 semaines ; les globules rouges sont éliminés par traitement à l'aide d'une solution de chlorure d'ammonium à 9 ‰ dans de l'eau, puis les cellules sont centrifugées à 600 *g* pendant 10 minutes et remises en suspension dans du PBS, avant d'être comptées et injectées.

### 2) Isolement des cellules du stroma vasculaire (stroma-vascular fraction ou SVF)

Les cellules sont isolées selon le protocole décrit par Bjômtorp et al., précité. De façon plus précise, le tissu adipeux inguinal est prélevé chez des souris C57B1/6 mâle âgées de 6 semaines et digéré à 37°C pendant 45 min, dans du tampon PBS contenant 0,2 % de BSA et 2 mg/ml de collagénase. Le produit de digestion est filtré successivement sur un filtre de 100 µm et de 25 µm, puis il est centrifugé à 800 g pendant 10 minutes ; les cellules stromales ainsi isolées sont remises en suspension dans du tampon PBS, puis elles sont comptées et utilisées dans des expériences de transplantation ou pour des analyses immunologiques.

### 3) Culture de la lignée préadipocytaire

La lignée de préadipocytes de souris 3T3-L1 (ATCC référence CL-173) est cultivée dans du milieu DMEM contenant 10 % de sérum de veau foetal inactivé par la chaleur et 2 mM de L-glutamine. Les cultures de cellules 3T3-L1 confluentes sont récoltées par trypsination, comptées et utilisées pour des expériences de transplantation ou des analyses immunologiques.

### 4) Transplantation de cellules (moelle osseuse, stroma vasculaire et lignée 3T3-L1)

Le jour de la transplantation, des souris C57 B1/6 femelles âgées de 8 à 10 semaines reçoivent une irradiation létale de 10 Gy, en une seule dose, puis une injection de 5.10⁶ à 10⁷ cellules, dans un volume de 400 µl, par voie intraveineuse dans la veine de la queue ou par voie intrapéritonéale. Les souris sont alimentées avec de l'eau acidifiée et de la nourriture autoclavée. Les animaux sont manipulés conformément aux directives relatives à l'expérimentation animale.

### 5) Analyse hématologique

4, 8 ou 10 semaines après la transplantation, un échantillon de 200 µl de sang périphérique est prélevé dans le plexus rétro-orbital des souris transplantées et immédiatement transféré dans un tube contenant de l'héparine. Des échantillons de sang périphérique prélevés chez des souris non-irradiées sont utilisés comme témoin positif et des échantillons de sang périphérique prélevés chez des souris irradiées non-reconstituées sont utilisés comme témoin négatif. Le comptage des cellules sanguines totales et la proportion des différents types de cellules nucléées est effectué automatiquement par un appareil d'analyse hématologique.

### 6) Analyse par réaction de polymérisation en chaîne (PCR)

10 semaines après la transplantation, l'ADN génomique total est extrait des cellules de tissus hématopoïétiques (moelle osseuse, rate, thymus, foie) et du sang, selon les techniques classiques décrites dans Current Protocols in Molecular Biology, (1990-2000), John Wiley and Sons, Inc. New York. Les échantillons d'ADN sont amplifiés dans un volume de 50 µl contenant 20 pmoles de chacune des amorces du gène *sry,* spécifique du chromosome Y, selon le protocole décrit dans Pang et al., précité.

A partir de l'ADN des souris transplantées avec des cellules de moelle osseuse ou de cellules du stroma vasculaire, on obtient un fragment de 722 pb, correspondant aux positions 256 à 978 du gène *sry*.

A partir de l'ADN des souris transplantées avec la lignée 3T3-L1, on obtient différents fragments qui ne correspondent pas au gène *sry* mais dont le profil est spécifique de ces cellules. '

Pour chaque série d'amplification, des échantillons provenant de souris mâles et femelles sont utilisés respectivement, comme témoin positif et négatif.

### 7) Analyse immunochimique

Les cellules en suspension, isolées comme décrit à l'exemple 1.2, sont incubées avec un premier anticorps, anti-CD34 couplé à la biotine (Clinisciences) ou anti-CD45 (Clinisciences), dilué dans du Tampon PBS contenant 0,1 % de BSA. Après des lavages et des centrifugations, les cellules sont incubées respectivement avec un anticorps secondaire [anti-immunoglobulines de souris couplées à l'isothyocyanate de fluorescéine (A2COL6), anti-immunoglobulines de rat couplées au rouge de Texas (CD45)] et avec de la streptavidine couplée au Cy-chrome, selon les protocoles classiques décrits dans *Current Protocols in Molecular Biology,* précité. Puis, les cellules sont fixées dans du tampon PBS contenant 0,037% de para-formaldéhyde et analysées par cytométrie de flux, ou bien elles sont fixées sur des lamelles par centrifugation et observées en microscopie de fluorescence.

### 8) Différentiation hématopoïétique

Les progéniteurs ou précurseurs hématopoïétiques à court terme sont analysés à partir des tissus hématopoïétiques des souris C57 B1/6 femelles, irradiées de façon létale, puis transplantées selon le protocole décrit à l'exemple 1.4.

Les progéniteurs ou précurseurs hématopoïétiques à long terme sont analysés à partir des tissus hématopoïétiques de souris SCID ayant reçu une irradiation non-létale de 4 Gy, puis transplantées selon le protocole décrit à l'exemple 1.4..

### a) lignées lymphoïdes

Les thymocytes (précurseurs de lymphocytes) sont purifiés à partir du thymus des souris femelles transplantées, selon les techniques classiques telles que décrites dans Current protocols in Immunology (John E. Coligan, 2000, Wiley and son Inc, Library of Congress, USA). L'ADN génomique total est ensuite extrait de ces thymocytes et amplifié comme décrit à l'exemple 1.6.

### b) lignées myéloïdes

Des extraits de cellules de moelle osseuse et de rate des souris transplantées sont préparés selon les techniques classiques telles que décrites dans Current protocols in Immunology (John E. Coligan, 2000, Wiley and son Inc, Library of Congress, USA), puis les cellules sont ensemencées dans un milieu contenant 1% méthylcellulose, 15 % sérum de veau foetal, 1 % de sérum de boeuf, 10 µg/ml d'insuline humaine, 200 µg/ml transferrine, 10⁻⁴ M mercaptoéthanol, 2 mM L-glutamine, 50 ng/ml SCF murine recombinante, 10 ng/ml IL3 murine recombinante, 10 ng/ml IL6 humaine recombinante (milieu METHOCULT^{™} GF M3534, STEM CELL TECHNOLOGIES INC), selon les instructions du fabricant.

### 9) Différenciation musculaire

Des cellules du stroma vasculaire, isolées comme décrit à l'exemple 1.2 sont mises en culture dans le milieu semi-solide à base de méthylcellulose tel que défini-ci dessus (exemple 1.8).

Les cardiomyocytes et les cellules musculaires squelettiques sont détectées par l'expression de l'α-actinine qui est mise en évidence à l'aide d'anticorps spécifiques (clone EA-53, SIGMA), selon les instructions du fabricant.

Les cellules musculaires squelettiques sont détectées spécifiquement par l'expression de la chaîne lourde de l'isoforme de myosine (isoformes rapides) qui est mise en évidence à l'aide d'anticorps spécifiques (clone MY-32, SIGMA), selon les instructions du fabricant.

Les cardiomyocytes sont également détectés par leur activité contractile spontanée, en présence ou en l'absence d'agonistes ou d'antagonistes des récepteurs muscariniques de l'acétylcholine (carbamylcholine et atropine, respectivement) ou β-adrénergiques (isoproténérol et propranolol, respectivement).

De manière plus précise, 1 ml de milieu DMEM contenant de la carbamylcholine (2µM), de l'atropine (10 µM), de l'isoproténérol (10 µM) ou du propranolol (40 µM) est ajouté sur le milieu à base de méthylcellulose. Après une incubation de 5 min nécessaire à la diffusion des molécules, le tampon excédentaire est éliminé et les contractions des cellules sont comptées sous le microscope pendant une minute.

### EXEMPLE 2 : Reconstitution des lignées hématopoïétiques à partir de cellules de moelle osseuse (contrôle) ou de cellules du stroma vasculaire, chez des souris femelles irradiées de façon létale.

Les souris receveuses sont irradiées puis transplantées par voie intrapéritonéale, avec des cellules du stroma vasculaire ou bien avec des cellules de moelle osseuse (contrôle), selon les protocoles décrits à l'exemple 1.

### 1) Survie des animaux irradiés

La figure 1A illustre la survie des animaux analysée 10 semaines après l'irradiation, de façon à évaluer la reconstitution durable des lignées hématopoïétiques. Les résultats observés montrent que les souris non-reconstituées meurent dans les 3 semaines suivant l'irradiation. En revanche, une survie de 40 % est observée chez les animaux transplantées avec des cellules du stroma vasculaire ou bien avec des cellules de moelle osseuse. Etant donné que l'irradiation létale élimine la majorité des précurseurs hématopoïétiques endogènes, les résultats observés indiquent que la survie des animaux reconstitués est liée aux cellules greffées.

### 2) Analyse des lignée hématopoiëtiques

Les figures 1B et 1C illustrent la reconstitution des différentes lignées hématopoïétiques, exprimée en pourcentage par rapport aux valeurs du témoin non-irradié.

Chez les souris non-reconstituées, le nombre de plaquettes chute rapidement en une semaine, d'une valeur initiale de 551. 10³ plaquettes/µl, à une valeur de 145 ± 6. 10³ plaquettes/µl. En revanche, chez les souris transplantées avec des cellules du stroma vasculaire ou des cellules de moelle osseuse, le nombre de plaquettes augmente progressivement pour atteindre des valeurs significatives à 4 semaines qui rejoignent quasiment celles du témoin à 10 semaines (figure 1C).

Chez les souris reconstituées par les cellules du stroma vasculaire, les leucocytes sont presque indétectables une semaine après l'irradiation mais 7 semaines plus tard, ils retrouvent des valeurs identiques à celle du témoin.

Les figures 1B et 1C montrent également que la restauration du nombre de plaquettes et de leucocytes est plus rapide chez les souris reconstitués avec les cellules de moelle osseuse.

L'analyse de la population de leucocytes montre que chez les souris reconstitués par des cellules de moelle osseuse ou des cellules du stroma vasculaire, les proportions de lymphocytes, de monocytes et de granulocytes sont équivalentes à celles des souris témoin non-irradiées.

Par conséquent, ces résultats démontrent que l'injection intrapéritonéale de cellules du stroma vasculaire permet de maintenir en vie des souris irradiées de façon létale et permet la reconstitution des lignées myéloïdes et lymphoïdes avec une efficacité comparable à celle observée avec un nombre équivalent de cellules de moelle osseuse, mais avec un retard de quelques semaines.

### 3) Mise en évidence des cellules greffées chez les souris receveuses

La présence de cellules mâles dérivées des cellules injectées dans les tissus hématopoïétiques et le sang des souris femelles reconstituées a été analysée par PCR à l'aide d'amorces du gène *sry,* spécifique du chromosome Y.

Aucune cellule mâle n'est détectée dans le groupe de souris non-reconstituées ou chez les souris femelles témoin.

En revanche, un produit de 722 pb spécifique du gène *sry* est présent en quantité très importante, dans les tissus hématopoïétiques (moelle osseuse, thymus, rate) et dans le sang des souris injectées avec des cellules de moelle osseuse issues de souris mâles (figure 2). Un produit de taille identique est également détecté dans les tissus hématopoïétiques (moelle osseuse, thymus, rate) et dans le sang des souris femelles, 10 semaines après la transplantation de cellules du stroma vasculaire, issues de souris mâles (figure 2).

Par conséquent, les résultats présentés à la figure 2 montrent que certaines cellules du stroma vasculaire ont la capacité de migrer de la cavité péritonéale vers les sites hématopoïétiques, de proliférer et de se différencier en cellules sanguines circulantes, permettant ainsi de reconstituer une hématopoïèse fonctionnelle chez les souris irradiées de façon létale.

### 4) Analyse de la différenciation hématopoïétique

Le gène *sry* est détecté dans les thymocytes (précurseurs de lymphocytes) purifié, issus des souris femelles transplantées avec des cellules du stroma vasculaire du tissu adipeux de souris mâle, indiquant que ces cellules ont un potentiel de différenciation en lignées lymphoïdes.

Des clones de cellules myeloïdes contenant le gène *sry* sont obtenues à partir des cellules de la moelle osseuse et de la rate des souris (C57 B1/6 et SCID) transplantées avec des cellules du stroma vasculaire du tissu adipeux ; chez les souris SCID irradiées de façon non létale, le nombre de clones hématopoïétiques contenant le gène *sry* est significativement supérieur. Ces résultats indiquent que les cellules du stroma vasculaire du tissu adipeux contiennent des progéniteurs hématopoïétiques capables de se différencier en lignées myéloïdes.

L'ensemble de ces résultats indique que le stroma vasculaire du tissu adipeux contient des progéniteurs hématopoïétiques à court terme et à long terme capables de se différencier en lignées hématopoïétiques lymphoïdes et myeloïdes, aptes à reconstituer une hématopoïèse fonctionnelle chez les souris irradiées.

### EXEMPLE 3 : Analyse phénotypique des cellules du stroma vasculaire.

Dans la mesure où le stroma vasculaire est constitué d'une population cellulaire hétérogène, des expériences d'immunomarquage en cytométrie de flux ont été réalisées afin d'identifier les cellules du stroma vasculaire qui possèdent une activité hématopoïétique.

La figure 3 montre que 35,3 ± 3,6 % de la population de cellules du stroma vasculaire expriment l'antigène A2COL6 qui est un marqueur spécifique des préadipocytes (panneau 2). En utilisant 2 antigènes spécifiques des cellules souches hématopoïétiques, des expériences d'immunomarquage indiquent que respectivement 35,8 ± 6 % et 30,6 ± 3,1 % des cellules sont positives pour CD34 et CD45, ce qui démontre que les cellules du stroma vasculaire, isolées à partir du tissus adipeux sont une source inattendue de cellules souche hématopoïétiques aptes à se différencier en cellules des différentes lignées hématopoïétiques (lignées myéloïdes et lymphoïdes).

Des expériences complémentaires de triple marquage révèlent que la majorité des cellules A2COL6 positives expriment également les antigènes CD34 et CD45 (figure 3 : panneaux 3 et 4) ce qui démontre que les préadipocytes peuvent être considérés comme des précurseurs hématopoïétiques. Ce triple marquage a également été obtenu avec la lignée préadipocytaire 3T3-L1.

### EXEMPLE 4 : Reconstitution des lignées hématopoïétiques à partir de cellules de la lignée préadipocytaire 3T3-L1, chez des souris femelles irradiées de façon létale.

Les souris receveuses sont irradiées puis transplantées par voie intraveineuse ou intrapéritonéale, avec des cellules de la lignée préadipocytaire 3T3-L1, selon les protocoles décrits à l'exemple 1.

Des expériences préliminaires ont montré que les greffes sont moins efficaces lorsque les cellules sont injectées par voie intrapéritonéale, vraisemblablement en raison de la lenteur de la migration des cellules, de la cavité péritonéale, vers les centres hématopoïétiques.

Par conséquent les résultats des injections intraveineuses sont présentés à la figure 4.

La figure 4A montre que 10 semaines après l'irradiation létale, 80 % des souris transplantées avec des cellules de moelle osseuse sont toujours vivantes alors que seulement 50 % des souris transplantées avec des cellules 3T3-L1 ont survécu à l'irradiation.

La numération des cellules sanguines des deux groupes de souris transplantées montre une restauration partielle du nombre de plaquettes et de leucocytes dans les 4 semaines suivant l'irradiation (figure 4B et 4C). A 10 semaines le nombre de plaquettes retrouve des valeurs équivalentes à celles des témoins non-irradiés (figure 4B) pour les deux groupes de souris transplantées. En revanche, à 10 semaines les souris reconstituées avec des cellules de moelle osseuse retrouvent des valeurs équivalentes à celles des témoins non-irradiés, alors que le nombre de leucocytes ne dépasse pas 50 % des valeurs des témoins chez les souris reconstituées avec des cellules 3T3-L1.

L'analyse de la population de leucocytes montre que chez les souris reconstituées par des cellules 3T3-L1, les proportions de lymphocytes, de monocytes et de granulocytes sont équivalentes à celle des souris témoins non-irradiées.

Par conséquent, ces résultats démontrent que comparativement aux cellules de moelle osseuse qui sont plus efficaces et permettent la reconstitution des lignées myéloïdes et lymphoïdes avec des valeurs comparables à celles des témoins non-irradiés, dès 8 semaines après l'irradiation, la lignée 3T3-L1 permet cependant une reconstitution partielle des lignées hématopoïétiques.

### EXEMPLE 5: Différenciation in vitro, de cellules du stroma vasculaire du tissu

### adipeux en cellules musculaires cardiaques et squelettiques

Des cellules du stroma vasculaire, isolées comme décrit à l'exemple 1.2 sont mises en culture puis analysées dans les conditions décrites à l'exemple 1. 9.

Dans ces conditions, on observe une multiplication des cellules puis une différenciation des cellules en cellules contractiles cardiaques et squelettiques.

La figure 5 montre la présence de cardiomyocytes et de cellules musculaires squelettiques caractérisées par l'expression de l'α-actinine. Elle montre également la détection spécifique des cellulaires musculaires squelettiques par l'expression de la chaîne lourde de l'isoforme de la myosine.

Le Tableau I ci-dessous montre l'activité contractile spontanée des cellules qui est spécifique des cardiomyocytes, ainsi que l'inhibition des contractions par le carbamylcholine (agoniste des récepteurs muscariniques à l'acétylcholine) et l'inversion de son effet par l'addition d'atropine (antagoniste des mêmes récepteurs). Les valeurs correspondent à la moyenne de 3 mesures indépendantes.

**Tableau I**

| | | | |
|---|---|---|---|
| Carbamylcholine | - | + | + |
| Atropine | - | - | + |
| Contractions | 100% | 53% | 106% |

Le Tableau II ci-dessous montre la stimulation de la fréquence des contractions par l'isoprotérénol (agoniste des récepteurs β-adrénergiques) et l'inversion de son effet par l'addition de propranolol (antagoniste des mêmes récepteurs). Les valeurs correspondent à la moyenne de 3 mesures indépendantes

**Tableau II**

| | | | |
|---|---|---|---|
| Isoproténérol | - | + | + |
| Propranolol | - | - | + |
| Contractions | 100% | 160% | 100% |

## Revendications

1. Utilisation de la fraction cellulaire du stroma vasculaire du tissu adipeux extra-médullaire, pour la préparation d'un médicament destiné au traitement des cardiomyopathies et des maladies dans lesquelles on observe une dégénérescence musculaire cardiaque.

2. Cellules isolées et purifiées, aptes à se différencier en cardiomyocytes, **caractérisées en ce qu'**elles sont susceptibles d'être obtenues par les étapes successives suivantes :
- prélèvement d'un échantillon de tissu adipeux extra-médullaire,
- isolement de la fraction cellulaire du stroma vasculaire,
- culture des cellules dans un milieu semi-solide contenant des facteurs de croissance et/ou des cytokines appropriées et
- purification des cellules par séparation physique et/ou par immunosélection, et
**en ce qu'**elles expriment au moins un marqueur des précurseurs adipocytaires sélectionné dans le groupe constitué par A2COL6/pOb24 et Pref-1, et au moins un marqueur des précurseurs cardiomyocytaires sélectionné dans le groupe constitué par l'α-actinine et le facteur GATA-4.

3. Cellules selon la revendication 2, **caractérisées en ce que** ledit milieu semi-solide contenant des facteurs de croissance et/ou des cytokines appropriées est un milieu contenant de la méthylcellulose additionnée de sérum de veau foetal, de sérum de boeuf, d'insuline, de transferrine, de SCF (*Stem Cell Factor*), d'IL3 et d'IL6.

4. Cellules selon la revendication 2 ou la revendication 3, **caractérisées en ce que** l'isolement de la fraction cellulaire du stroma vasculaire s'effectue par digestion de la matrice extracellulaire par des enzymes protéolytiques et par séparation physique.

5. Cellules selon l'une quelconque des revendications 2 à 4, **caractérisées en ce que** la séparation physique est réalisée par différence d'adhésion sur un support solide approprié.

6. Cellules selon l'une quelconque des revendications 2 à 5, **caractérisées en ce qu'**elles expriment au moins A2COL6/pOb24.

7. Cellules selon l'une quelconque des revendications 2 à 6, **caractérisées en ce qu'**elles sont d'origine humaine.

8. Cellules modifiées, **caractérisées en ce qu'**elles sont constituées par des cellules selon l'une quelconque des revendications 2 à 6 génétiquement modifiées.

9. Cellules modifiées selon la revendication 8, **caractérisées en ce qu'**elles comprennent au moins une mutation d'un gène autologue.

10. Cellules modifiées selon la revendication 8, **caractérisées en ce qu'**elles contiennent au moins une copie d'un gène hétérologue.

11. Cellules selon l'une quelconque des revendications 8 à 10, **caractérisées en ce qu'**elles sont d'origine humaine.

12. Lignées de cellules immortalisées issues des cellules humaines selon la revendication 7 ou la revendication 11.

13. Médicament destiné à la reconstruction du myocarde, **caractérisé en ce qu'**il comprend des cellules selon l'une quelconque des revendications 2 à 7 ou bien des cellules modifiées ou des lignées issues de ces cellules selon l'une quelconque des revendications 8 à 12 et au moins un véhicule pharmaceutiquement acceptable.

14. Utilisation des cellules selon l'une quelconque des revendications 2 à 7 ou bien des cellules modifiées ou des lignées issues de ces cellules selon l'une quelconque des revendications 8 à 12 pour la préparation d'un médicament destiné au traitement des cardiomyopathies et des maladies liées à une dégénérescence musculaire cardiaque.

15. Procédé de préparation de cellules isolées et purifiées aptes à se différencier en cardiomyocytes, telles que définies à l'une quelconque des revendications 2 à 7, lequel procédé est **caractérisé en ce qu'**il comprend au moins les étapes suivantes :
a) prélèvement d'un échantillon de tissu adipeux extra-médullaire,
b) isolement de la fraction cellulaire du stroma vasculaire, de préférence, par digestion de la matrice extracellulaire par des enzymes protéolytiques, et par séparation physique,
c₁) culture des cellules dans un milieu semi-solide contenant des facteurs de croissance et/ou des cytokines appropriées, et
c₂) purification des cellules par séparation physique et/ou par immunosélection.

16. Procédé selon la revendication 15 **caractérisé en ce qu'**il comprend une étape supplémentaire d) d'expansion des cellules *in vitro.*

17. Procédé selon la revendication 15 ou la revendication 16, **caractérisé en ce que** l'immunosélection est réalisée à l'aide d'un anticorps spécifique d'un marqueur exprimé par lesdites cellules tel que défini à la revendication 2.

18. Utilisation des cellules selon l'une quelconque des revendications 2 à 7 ou bien des cellules modifiées ou des lignées issues de ces cellules selon l'une quelconque des revendications 8 à 12 pour le criblage de molécules capables de moduler l'activité musculaire cardiaque.

19. Procédé de préparation de cardiomyocytes, **caractérisé en ce qu'**il comprend les étapes suivantes :
a) culture de cellules purifiées et isolées à partir de la fraction cellulaire du stroma vasculaire du tissu adipeux extra-médullaire, dans un milieu semi-solide contenant des facteurs de croissance et/ou des cytokines appropriées, et
b) sélection des cellules qui présentent une activité contractile spontanée.

20. Procédé selon la revendication 19, **caractérisé en ce que** l'étape b) comprend la sélection desdites cellules dont l'activité contractile est inhibée de façon réversible par un agoniste des récepteurs muscariniques à l'acétylcholine.

21. Procédé selon la revendication 19, **caractérisé en ce que** l'étape b) comprend la sélection desdites cellules dont la fréquence de contraction est stimulée de façon réversible par un agoniste des récepteurs β-adrénergiques.

22. Procédé selon l'une quelconque des revendications 19 à 21, **caractérisé en ce que** ledit milieu semi-solide est de la méthylcellulose.

23. Procédé selon l'une quelconque des revendications 19 à 22, **caractérisé en ce que** lesdits facteurs de croissance et/ou cytokines appropriées sont le sérum de veau foetal, le sérum de boeuf, l'insuline, la transferrine, le SCF, l'IL3 et l'IL6.

## Patentansprüche

1. Verwendung der stroma-vaskulären Zellfraktion des extramedullären Fettgewebes zur Herstellung eines Medikaments zur Behandlung von Kardiomyopathien und Erkrankungen, bei denen eine Degeneration des Herzmuskels zu beobachten ist.

2. Isolierte und gereinigte Zellen, die zu Kardiomyozyten differenzieren können, **dadurch gekennzeichnet, dass** sie durch die folgenden aufeinanderfolgenden Schritte erhältlich sind:
- Entnahme einer Probe von extramedullärem Fettgewebe,
- Isolierung der stroma-vaskulären Zellfraktion,
- Kultivieren der Zellen in einem halbfesten Medium, das geeignete Wachstumsfaktoren und/oder Zytokine enthält, und
- Reinigung der Zellen durch physikalische Abtrennung und/oder durch lmmunselektion,
und dadurch, dass sie wenigstens einen Marker von Adipozyten-Vorläufern exprimieren, der ausgewählt ist aus der Gruppe bestehend aus A2COL6/pOb24 und Pref-1, und wenigstens einen Marker von Kardiomyozyten-Vorläufern, der ausgewählt ist aus der Gruppe bestehend aus α-Actinin und Faktor GATA-4.

3. Zellen nach Anspruch 2, **dadurch gekennzeichnet, dass** das halbfeste Medium, das geeignete Wachstumsfaktoren und/oder Zytokine enthält, ein Medium ist, das mit fötalem Kälberserum, Rinderserum, Insulin, Transferrin, SCF (Stammzellfaktor), IL3 und IL6 supplementierte Methylcellulose enthält.

4. Zellen nach Anspruch 2 oder Anspruch 3, **dadurch gekennzeichnet, dass** die Isolierung der stroma-vaskulären Zellfraktion durch Verdau der extrazellulären Matrix durch proteolytische Enzyme und physikalische Abtrennung erfolgt.

5. Zellen nach einem der Ansprüche 2 bis 4, **dadurch gekennzeichnet, dass** die physikalische Abtrennung durch unterschiedliche Adhäsion an einem geeigneten festen Träger erfolgt.

6. Zellen nach einem der Ansprüche 2 bis 5, **dadurch gekennzeichnet, dass** sie wenigstens A2COL6/pOb24 exprimieren.

7. Zellen nach einem der Ansprüche 2 bis 6, **dadurch gekennzeichnet, dass** sie humanen Ursprungs sind.

8. Modifizierte Zellen, **dadurch gekennzeichnet, dass** sie aus Zellen nach einem der Ansprüche 2 bis 6 gebildet sind, die genetisch modifiziert sind.

9. Modifizierte Zellen nach Anspruch 8, **dadurch gekennzeichnet, dass** sie wenigstens eine Mutation eines autologen Gens umfassen.

10. Modifizierte Zellen nach Anspruch 8, **dadurch gekennzeichnet, dass** sie wenigstens eine Kopie eines heterologen Gens enthalten.

11. Zellen nach einem der Ansprüche 8 bis 10, **dadurch gekennzeichnet, dass** sie humanen Ursprungs sind.

12. lmmortalisierte Zelllinien, die sich von Humanzellen nach Anspruch 7 oder Anspruch 11 ableiten.

13. Medikament zur Regeneration des Myokards, **dadurch gekennzeichnet, dass** es Zellen nach einem der Ansprüche 2 bis 7 oder modifizierte Zellen oder Zelllinien, die sich von diesen Zellen ableiten, nach einem der Ansprüche 8 bis 12 und wenigstens einen pharmazeutisch verträglichen Träger umfasst.

14. Verwendung von Zellen nach einem der Ansprüche 2 bis 7 oder von modifizierten Zellen oder von Zelllinien, die sich von diesen Zellen ableiten, nach einem der Ansprüche 8 bis 12 zur Herstellung eines Medikaments zur Behandlung von Kardiomyopathien und Erkrankungen, die mit einer Degeneration des Herzmuskels einhergehen.

15. Verfahren zur Präparation von isolierten und gereinigten Zellen, die zu Kardiomyozyten differenzieren können, gemäß einem der Ansprüche 2 bis 7, wobei das Verfahren **dadurch gekennzeichnet ist, dass** es wenigstens die folgenden Schritte umfasst:
a) Entnahme einer Probe von extramedullärem Fettgewebe,
b) Isolierung der stroma-vaskulären Zellfraktion, vorzugsweise durch Verdau der extrazellulären Matrix durch proteolytische Enzyme und durch physikalische Abtrennung
c₁) Kultivieren der Zellen in einem halbfesten Medium, das geeignete Wachstumsfaktoren und/oder Zytokine enthält, und
c₂) Reinigung der Zellen durch physikalische Abtrennung und/oder durch Immunselektion,

16. Verfahren nach Anspruch 15, **dadurch gekennzeichnet, dass** es einen zusätzlichen Schritt d) der Expansion der Zellen *in vitro* umfasst.

17. Verfahren nach Anspruch 15 oder Anspruch 16, **dadurch gekennzeichnet, dass** die lmmunselektion mittels eines Antikörpers erfolgt, der für einen Marker spezifisch ist, der von den Zellen gemäß Anspruch 2 exprimiert wird.

18. Verwendung von Zellen nach einem der Ansprüche 2 bis 7 oder von modifizierten Zellen oder von Zelllinien, die sich von diesen Zellen ableiten, nach einem der Ansprüche 8 bis 12 zum Screenen von Molekülen, die fähig sind, die Herzmuskelaktivität zu modulieren.

19. Verfahren zur Präparation von Kardiomyozyten, **dadurch gekennzeichnet, dass** es die folgenden Schritte umfasst:
a) Kultivieren von gereinigten und aus der stroma-vaskulären Zellfraktion des extramedullären Fettgewebes isolierten Zellen in einem halbfesten Medium, das geeignete Wachstumsfaktoren und/oder Zytokine enthält, und
b) Selektion der Zellen, die eine spontane kontraktile Aktivität zeigen.

20. Verfahren nach Anspruch 19, **dadurch gekennzeichnet, dass** Schritt b) die Selektion der Zellen umfasst, deren kontraktile Aktivität reversibel durch einen Agonisten muskarinischer Acetylcholinrezeptoren gehemmt wird.

21. Verfahren nach Anspruch 19, **dadurch gekennzeichnet, dass** Schritt b) die Selektion der Zellen umfasst, deren Kontraktionsfrequenz reversibel durch einen Agonisten β-adrenerger Rezeptoren stimuliert wird.

22. Verfahren nach einem der Ansprüche 19 bis 21, **dadurch gekennzeichnet, dass** das halbfeste Medium Methylcellulose ist.

23. Verfahren nach einem der Ansprüche 19 bis 22, **dadurch gekennzeichnet, dass** die geeigneten Wachstumsfaktoren und/oder Zytokine fötales Kälberserum, Rinderserum, Insulin, Transferrin, SCF, IL3 und IL6 sind.

## Claims

1. Use of the cellular fraction of the vascular stroma of extra-medullary adipose tissue for the preparation of a medicament intended for the treatment of cardiomyopathies and diseases in which cardiac muscle degeneration is observed.

2. Isolated and purified cells, suitable for differentiating into cardiomyocytes, **characterised in that** they can be obtained by the following successive steps:
- taking a sample of extra-medullary adipose tissue,
- isolating the cellular fraction of the vascular stroma,
- culturing the cells in a semi-solid medium containing appropriate growth factors and/or cytokines, and
- purifying the cells by physical separation and/or by immuno-selection, and
**in that** they express at least one marker for adipocyte precursors selected from the group comprising A2COL6/pOb24 and Pre-1, and at least one marker for cardiomyocyte precursors selected from the group comprising α-actinin and GATA-4 factor.

3. Cells as claimed in claim 2, **characterised in that** said semi-solid medium containing appropriate growth factors and/or cytokines is a medium containing methyl cellulose supplemented with foetal calf serum, bovine serum, insulin, transferrin, SCF (*Stem Cell Factor*), IL3 and IL6.

4. Cells as claimed in claim 2 or claim 3, **characterised in that** the cellular fraction of the vascular stroma is isolated by digestion of the extra-cellular matrix by proteolytic enzymes and by physical separation.

5. Cells as claimed in any one of claims 2 to 4, **characterised in that** the physical separation is effected on the basis of a difference in adhesion to an appropriate solid support.

6. Cells as claimed in any one of claim 2 to 5, **characterised in that** they express at least A2COL6/pOb24.

7. Cells as claimed in any one of claims 2 to 6, **characterised in that** they are of human origin.

8. Modified cells, **characterised in that** they are cells as claimed in any one of claims 2 to 6 that are genetically modified.

9. Modified cells as claimed in claim 8, **characterised in that** they comprise at least one mutation of an autologous gene.

10. Modified cells as claimed in claim 8, **characterised in that** they contain at least one copy of a heterologous gene.

11. Cells as claimed in any one of claims 8 to 10, **characterised in that** they are of human origin.

12. Immortalised cell lines derived from human cells as claimed in claim 7 or claim 11.

13. Medicament intended to regenerate the myocardium, **characterised in that** it comprises cells as claimed in any one of claims 2 to 7 or modified cells or lines derived from these cells as claimed in any one of claims 8 to 12 and at least one pharmaceutically acceptable vehicle.

14. Use of cells as claimed in any one of claims 2 to 7 or modified cells or lines derived from these cells as claimed in any one of claims 8 to 12 for the preparation of a medicament intended for the treatment of cardiomyopathies and diseases associated with cardiac muscle degeneration.

15. Method of preparing isolated and purified cells capable of differentiating into cardiomyocytes as defined in any one of claims 2 to 7, which method is **characterised in that** it comprises at least the following steps:
a) taking a sample of extra-medullary adipose tissue,
b) isolating the cellular fraction of the vascular stroma, preferably by digestion of the extra-cellular matrix by proteolytic enzymes and by physical separation,
c₁) culturing the cells in a semi-solid medium containing appropriate growth factors and/or cytokines, and
c₂) purifying the cells by physical separation and/or by immuno-selection.

16. Method as claimed in claim 15, **characterised in that** it comprises an additional step d) of expanding the cells *in vitro.*

17. Method as claimed in claim 15 or claim 16, **characterised in that** the immuno-selection is effected with the aid of an antibody specific for a marker expressed by said cells as defined in claim 2.

18. Use of the cells as claimed in any one of claims 2 to 7 or modified cells or lines derived from these cells as claimed in any one of claim 8 to 12 for screening molecules capable of modulating cardiac muscle activity.

19. Method of preparing cardiomyocytes, **characterised in that** it comprises the following steps:
a) culturing isolated and purified cells from the cellular fraction of the vascular stroma of extra-medullary adipose tissue in a semi-solid medium containing appropriate growth factors and/or cytokines, and
b) selecting the cells which exhibit a spontaneous contractile activity.

20. Method as claimed in claim 19, **characterised in that** step b) comprises the selection of said cells, for which the contractile activity is reversibly inhibited by an agonist of muscarinic acetyl choline receptors.

21. Method as claimed in claim 19, **characterised in that** step b) comprises the selection of said cells for which the contraction frequency is reversibly stimulated by an agonist of β-adrenergic receptors.

22. Method as claimed in any one of claims 19 to 21, **characterised in that** said semi-solid medium is methyl cellulose.

23. Method as claimed in any one of claims 19 to 22, **characterised in that** said appropriate growth factors and/or cytokines are foetal calf serum, bovine serum, insulin, transferrin, SCF, IL3 and IL6.
